# EUROPEAN PATENT SPECIFICATION

(11) **EP 2 338 532 B1**
(45) Date of publication and mention of the grant of the patent: **10.01.2018**
(21) Application number: 10382243.3
(22) Date of filing: 10.09.2010
(51) Int. Cl.: A61L 27/18, A61L 27/56, C08J 9/26, C08J 9/28

(54) **Porous PEEK article as an implant**
Poröser PEEK-Gegenstand als ein Implantat
Article Peek poreux en tant qu'implant

(30) Priority: 23.12.2009 EP 09382298
(43) Date of publication of application: 29.06.2011
(73) Proprietor: Fundacion Inasmet, 20009 San Sebastian (ES)
(72) Inventor: Olalde Graells, Beatriz, 20009, SAN SEBASTIAN (ES); Jurado Oñate, María, Jesús, 20009, SAN SEBASTIAN (ES)
(74) Representative: Carpintero Lopez, Francisco

(56) References cited:
- WO-A2-2007/051307
- GB-A- 1 486 867
- US-A- 3 772 059

## Description

### FIELD OF THE INVENTION

The present invention relates to a new porous PEEK-type article presenting at least a trimodal pore distribution and to a process for its preparation which comprises using a porogen agent as well as a solvent for generating the porosity. The resulting porous article is well suited for medical implants among other applications.

### BACKGROUND OF THE INVENTION

PEEK biocompatible materials have been used in the state of the art for bone implant applications. Their use in other applications, such as a scaffold has jet not been possible due to its structural limitations, lack of porosity, and thus the impossibility of the PEEK materials of resembling the bone structure to facilitate their integration. In this sense it must be stated that for bone tissue engineering applications, scaffold parameters like pore size, porosity and surface area are widely recognized as very important and are not fulfilled at present by the known PEEK materials. Other architectural features for scaffolds, such as pore shape, pore wall morphology, and interconnectivity between pores are also suggested to be fundamental for cell seeding, migration, growth, mass transport, gene expression, and new tissue formation in three-dimensions.

PEEK porous materials have been achieved according to a variety of methods of the art which in general present some disadvantages, mainly an inadequate morphology to comply with the above mentioned requirements for its medical application.

In particular, it is well known in the prior art a method, as described in WO2007/051307, which comprises producing a porous article by mixing a salt-type porogen agent such as sodium chloride with a PEEK polymer to form a moulding material, which is then subjected to a moulding process to produce a moulded article and subsequently washing said article to leach the porogen agent, hereby forming pores. In a particular embodiment the PEEK presents a lower melting point than the porogen agent and the process comprises heating the mixture to a temperature between that of the melting point of the PEEK and that of the porogen agent, moulding and cooling the article until it solidifies. The so resulting material presents a pore size distribution resembling the pore size distribution of the porogen which does not provide the architectural features needed for bone regeneration.

According to this method the use of different porogen agents of different sizes has been contemplated, although this approach provides a porous structure with low connectivity between pores.

Other processes for obtaining porous PEEK materials are based on laser sintering such as the process disclosed in which require the use of high cost equipment (Tan, K. H. et al., Bio-Medical Materials and Engineering (2005), 15 (1,2) 113-124.

Known are as well processes as the one disclosed in JP 2006241363, based on molding by compression with a porogen agent which require high temperatures at which the PEEK polymer is molten, that is, temperatures are needed above the polymer melting point higher than 374°C.

Other methods are based on a thermally induced phase separation, which comprise the steps of dissolving a PEEK-type polymer in a polar organic solvent having a six-membered ring structure and a boiling point of 175°C to 420°C and casting the solution onto a support. Such a method is disclosed in EP 0 407 684 A1 and presents the disadvantage that the pore size is not greater than 10 µm.

The patent application GB1486867 describes a porous sheet; the porous sheet is made by forming a plastic mass of a thermoplastic polymer, particulate removable filler and an extractable organic solvent or plasticizer, forming the mixture into a sheet, removing the solvent or plasticizer by extraction with a fluorinated hydrocarbon.

In spite of the variety of methods none of the materials obtained accordingly presents the adequate morphological and porosity characteristics which allow their successful application in bone tissue engineering.

Thus in view of the above there is still the need in the art to provide new biocompatible articles with improved characteristics relative to pore shape, pore wall morphology, and interconnectivity between pores, among others, which are alleged to be fundamental for cell seeding, migration, growth, mass transport, gene expression, and new tissue formation in three-dimensions, and can thus be successfully used in bone tissue engineering applications.

### BRIEF DESCRIPTION OF THE FIGURES

Figure 1: SEM micrographs in increasing degree of magnification of a porous article prepared as described in Example 1.
Figure 2: SEM micrographs of a porous article prepared as described in Example 2.
Figure 3: SEM micrograph and EDS spectrum of a porous article prepared as described in Example 2.
Figure 4: SEM micrographs of the porous article prepared as described in Example 3.
Figure 5: a diagram of the porous article of the invention, SEM images of the article and the pore distributions.

### DESCRIPTION OF THE INVENTION

In one aspect of the present invention refers to a process for the production of a porous article comprising a polyetheretherketone-type polymer structure, hereinafter also referred to as PEEK-type polymer, comprising the following steps:
a) contacting a PEEK-type polymer with a composition comprising at least an organic solvent,
b) heating at a temperature at which the PEEK-type polymer is dissolved,
c) adding at least a porogen agent, in an amount comprised between 50 to 90% wt in respect of the mixture PEEK-type polymer-solvent weight,
d) cooling the mixture obtained in c) at a temperature at least equal or lower than the temperature at which the PEEK-type polymer precipitates,
e) forming said cooled mixture into a shaped intermediate article,
f) removing the organic solvent and the porogen agent,
g) recovering the article comprising a PEEK-type polymer.

The process, hereinafter the process of the invention provides after step e) an intermediate article comprising at least a PEEK-type polymer, a porogen agent, and an organic solvent. The organic solvent and the porogen agent are then removed in step f) and a porous article is recovered in g). This new PEEK-type porous article presents a new and very characteristic morphology which makes it very suitable for applications such as tissue engineering.

Said PEEK-type porous article, which constitutes another aspect of the present invention, comprises a PEEK-type polymer structure (or matrix) and presents at least a trimodal pore distribution as follows:
(i). - a pore distribution A corresponding to pores of an average size between 50 µm and 500 µm which are interconnected throughout the whole article, and are hereinafter also referred to as pores A;
(ii). - a pore distribution B corresponding to the voids between adjacent pores A of an average size between 5 µm and 70 µm; and are hereinafter also referred to pores B;
(iii). - a pore distribution C corresponding to pores of an average size of 5 µm or less corresponding to pores which are located in the walls of the pores A and pores B, hereinafter referred to as pores C.

The pore distribution A is generated in the process of the invention in step f) when the porogen agent is removed leaving the pores A which retain the shape of the porogen agent, and which are located within and throughout the whole PEEK-type polymer structure. The pores B of the pore distribution B are originated since in the shaped intermediate article obtained in step e), the porogen agent particles are adjacent and in contact. Thus, when the particles are removed, they leave voids between the adjacent pores A. The pore distribution C is generated due to the presence of the solvent in the intermediate article. When the solvent is then eliminated in step f) it leaves pores around 5 µm or smaller, which will be referred hereinafter as pores C. These pores C can be in the nanometric range or both in the micro- and nanometric ranges.

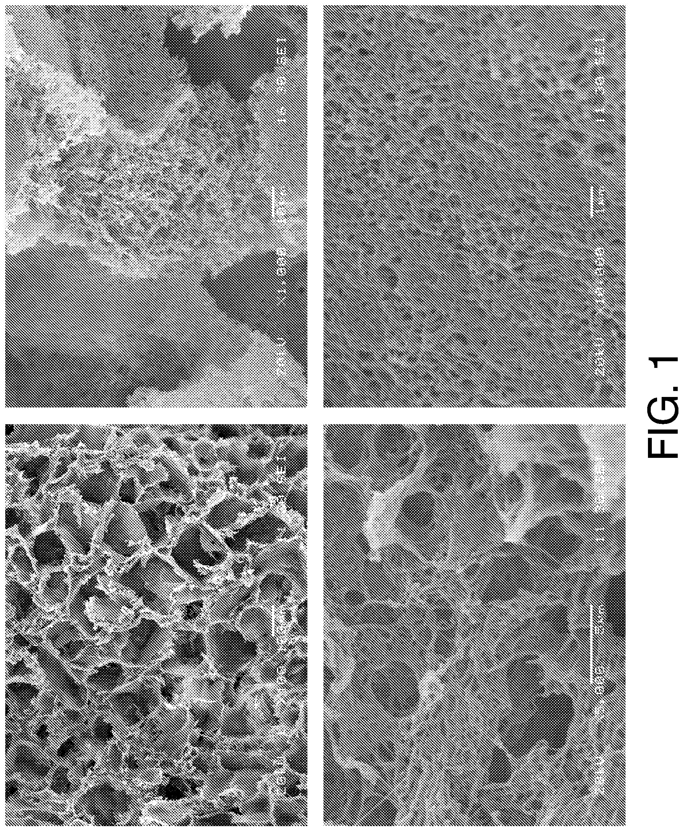

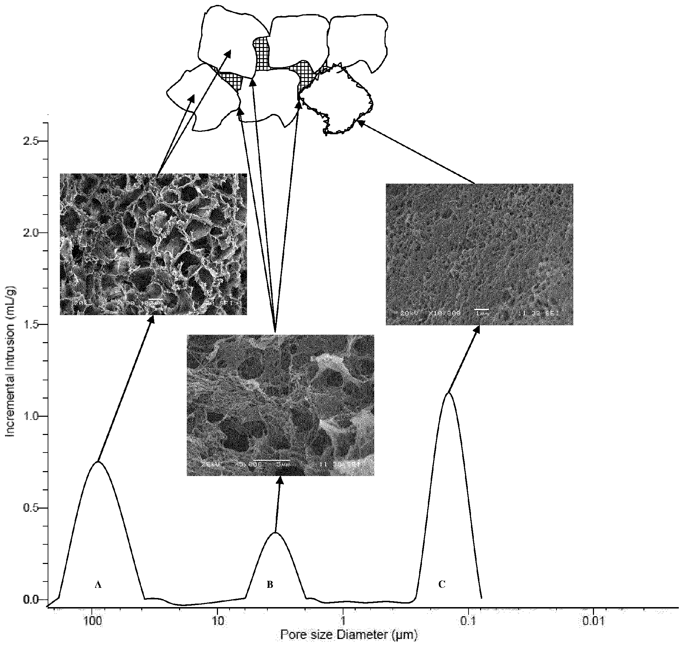

These three pore distributions can be clearly observed in Figures 1, 2 and 4. In Figure 5 a typical pore distribution is represented where it can be clearly seen that the pore distribution A (see **A**), centered at about 100 µm corresponds to the pores A, the pore distribution B (see **B**), centered between 7-8 µm, corresponds to the pores B (voids between adjacent pores A) and the pore distribution C (see **C**), centered at about 0.2 µm, corresponds to the pores C.

This characteristic morphology, comprising said at least trimodal pore distribution and different pore sizes is achieved according to the process of the invention by means of combining a porogen agent and an organic solvent. Thus, the process of the invention provides improved PEEK-type polymer articles which can, among other applications, be used as scaffolds in tissue engineering applications as it is further below disclosed in detail.

Polyetheretherketone-type (PEEK-type) polymers refer to polymers containing predominantly ether, -R-O-R-, and ketone, -R-CO-R-, linkages, wherein R is a divalent aromatic group. R is preferably a substituted or unsubstituted phenylene of the following Formula: 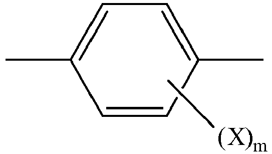 wherein
X is independently in each occurrence hydrogen, a C₁₋₄ alkyl, or a halogen; and
m is an integer between 0 and 4 inclusive.
X is preferably hydrogen, methyl, ethyl, chlorine, bromine, or fluorine.

Examples of poly(etheretherketone)-type polymers within the scope of this invention include poly(etherketone) (PEK), poly(aryletherketone) (PAEK), poly(etheretherketone) (PEEK), poly(etheretherketoneketone) (PEEKK), poly(etherketoneetherketoneketone) (PEKEKK), and mixtures thereof. A specially preferred poly(etheretherketone)-type polymer for use in this invention is PEEK, that is, poly(oxy-p-phenyleneoxy-p-phenylenecarbonyl-p-phenylene). PEEK is comprised of the repeat units described in the following Formula: 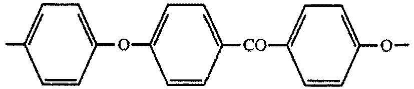 (PEEK-type) polymers for use in this invention are commercially available and/or can be obtained by synthesis processes well known in the art (US 4,320,224 and US 4,331,798).

The PEEK-type polymer is contacted with a composition comprising at least an organic solvent.

The organic solvent for use in the present invention may be a single solvent or a mixture of solvents. The solvent has to dissolve the PEEK-type polymer and has to present an appropriate boiling point. The selection of the solvent depends on the nature and the amount of the PEEK-type polymer used, and might be readily selected by the skilled person. The solvent used should not dissolve the porogen agent at the temperatures of the present process.

Solvents useful for making a solution of PEEK-type polymers are organic compounds with some degree of polarity. A large percentage of such organic compounds have an aromatic or polynuclear aromatic component. The solvents useful in this invention are organic compounds consisting predominantly of carbon and hydrogen and optionally oxygen, nitrogen, sulphur, halogen, and mixtures thereof, wherein the organic compound has typically a molecular weight of between 160 and 450. Each suitable solvent to be used in the present invention presents at least one six membered ring structure and a boiling point in a range between 150 °C and 400°C and is capable of dissolving at least 10% of the PEEK-type polymer present at the article forming temperature. The solvent preferably dissolves at the forming temperature at least 25 weight percent of the PEEK-type polymer, more preferably 50 weight percent of the PEEK-type polymer.

In a particular embodiment the organic solvent is selected from the group consisting of benzophenone, pentafluorophenol, phenilsulphone, 2-phenilphenol, dimethylphthalate, phenylbenzoate, ethyl-4-hydroxybezoate, n-cyclohexyl-2-pyrrolidone and mixtures thereof, preferably benzophenone or pentafluorophenol.

The composition comprising at least an organic solvent may further comprise a bioactive ceramic. In the context of the present invention a bioactive ceramic refers to a material capable of providing a specific biologic response in the material interface, which results from the union between material and tissues. A bioactive ceramic forms a union with adjacent tissues (Bauer TW, Smith ST: Bioactive materials in orthopaedic surgery. Overview and regulatory considerations. Clin Orthop 2002; 395: 11-22).

Bioactive ceramics useful in the present invention are any of the state of the art, such as for example, calcium phosphates, preferably tricalcium phosphate, hydroxyapatite, biphasic calcium phosphate, and their mixtures. The bioactive ceramics are used in the form of nanoparticles, microparticles or their mixtures.

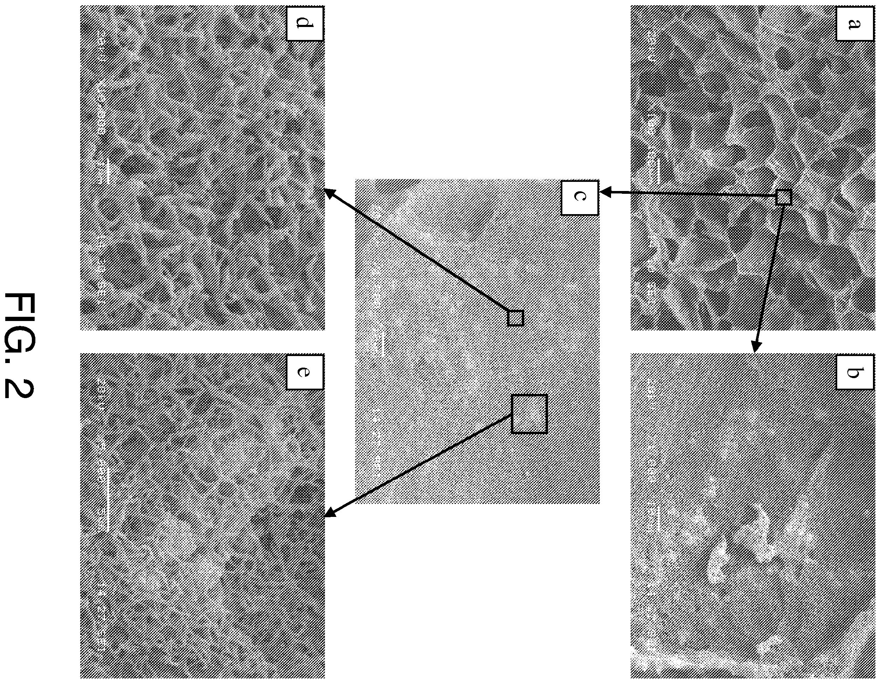

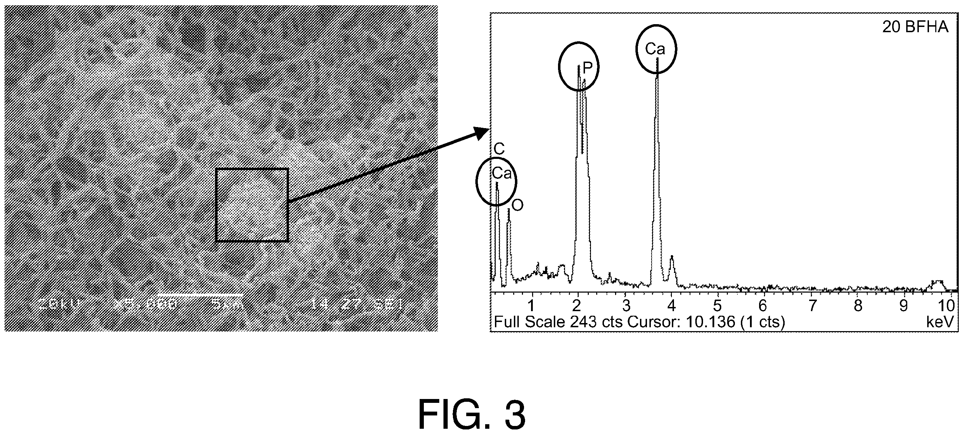

The inventors have shown that when bioactive ceramic particles are used (HA for instance with an average size of around 3 µm, see Example 2) said particles are distributed throughout the whole article (Figures 2b, c, e). These particles are successfully integrated in the PEEK-type polymer matrix with no visible agglomeration formation. In this sense it has also been shown from the EDS characterization (Figure 3) that the particles observed in the SEM images were, basically composed by calcium and phosphorous elements, two of the major components of HA.

In the process of the invention, heating is carried out while stirring the mixture of the PEEK-type polymer with a solvent or with a suspension comprising a solvent, whereupon, with stirring the PEEK-type polymer is dissolved in the solvent. Heating of the PEEK and the composition is carried out at a temperature typically comprised between 150°C and 400°C, although said temperature may vary depending on the amount of PEEK to be dissolved, its chemical nature, and the selected solvent. Heating is preferably carried out under inert atmosphere to prevent undesirable side reactions of any of the components. Nitrogen and argon are useful inert atmospheres.

The mixing times necessary to completely dissolve the polymer vary with the boiling point of the solvent chosen, and the temperature at which the mixture is heated. Said times may vary within a wide range, but are typically comprised between 30 to 120 minutes. The weight ratio of polymer to solvent can vary among a broad range. Typically the weight ratio of polymer to solvent is comprised between 5-50 weight percent. The weight ratio of bioactive ceramic to polymer can vary also among a broad range. Typically the weight ratio of bioactive ceramic to polymer is comprised between 5-50 weight percent

The steps a) and b) are carried out in a slightly different manner depending on the presence or not of a bioactive ceramic in the composition. Thus, according to a particular embodiment the PEEK-type polymer is contacted with a composition consisting of a selected solvent and the mixture is heated at a temperature at which the polymer is dissolved generally between 150°C and 400°C. Heating is carried out under inert atmosphere for the same reason as exposed above while stirring the mixture and the mixture of PEEK and solvent is stirred until complete dissolution is achieved rendering a homogenous and transparent solution.

In another embodiment a composition of a bioactive ceramic and a selected solvent is previously obtained consisting of a dispersion which is obtained under stirring and under inert atmosphere. The PEEK-type polymer is then contacted with the resulting dispersion and the obtained mixture is then heated, under stirring, whereupon, with stirring the PEEK-type polymer is dissolved in the solvent.

After the PEEK-type polymer is completely dissolved a porogen agent is added thereto. Said porogen agent may be organic or inorganic.

As the type of solvent and the amount of polymer is influencing the working temperature (which is generally between 150 and 400°C), it is necessary to use a porogen agent which is not soluble in the solvent used and does not melt at the working temperature. Therefore, the porogen agent is selected depending on the solvent used, and the working temperature.

In a particular embodiment the agent is selected from the group of sugar-type porogen agents, salt-type porogen agents and their mixtures. Preferred are salt-type porogen agents. Some exemplary salt-type porogen agents suitable for use in the present invention are sodium chloride, sodium citrate, sodium tartrate, potassium chloride, sodium fluoride, potassium fluoride, sodium iodide, sodium nitrate, sodium sulphate, sodium iodate, and mixtures thereof, preferably sodium chloride, sodium citrate, sodium tartrate, potassium chloride and more preferably sodium chloride or potassium chloride are used due to its availability and low cost. Some exemplary sugar-type porogen agents suitable for use in the present invention are water soluble sugars, for instance, sacarose, galactose, saccharine, glucose, fructose and their mixtures, preferably sacarose, galactose and their mixtures.

It is to be understood that the porogen agent materials are particles which can be formed in any shape and size as necessary, or desired, such as cubic, spheres, regular geometric shapes, irregular geometric shapes, and mixtures thereof. The porogen agent average particle size can be typically comprised between 50-500µm, and is selected depending on the desired porosity, pore size and form, and pore size distribution to be obtained.

The porogen agent is generally used in the invention in an amount comprised between 50 to 90%wt in respect of the mixture PEEK-type polymer - solvent weight.

According to a particular embodiment at least two different porogen agents, not necessarily differing in their chemical nature, but differing at least in their particle size distributions are used.

In step d) the mixture obtained in c) is cooled at a temperature at least equal or lower than the temperature at which the PEEK-type polymer precipitates. Said temperature at which the polymer solution becomes turbid, depends on the solvent and the polymer amount. At said temperature the mixture is then formed (step e)) into a shaped solidified intermediate article. Said temperature is generally the ambient temperature, which is maintained for a period a time typically comprised between several minutes and several hours until the mixture is formed. In a particular embodiment forming is done overnight. The size of the obtained pores C will also depend on said temperature, in such a way that the lower the temperature is, the smaller the pore C size.

The cooling and forming of the article may be made according to well known different methods from the state of the art depending for instance on the configuration (shape, dimension and size) of the article to be obtained. Forming the article in the present invention refers to the shaping of the hot mixture into the desired configuration.

According to a particular embodiment the mixture obtained in c) is casted at room temperature onto a supporting surface, such as a glass plate. The article thus finally obtained is then a 2D porous sample.

According to a preferred embodiment, forming the cooled mixture is carried out by placing said mixture in a mould presenting the shape and dimensions of the article to be obtained. Said mould can be of any conventional material, such as a glass vial, a metallic vial, or a Teflon vial for example.

After steps d) and e) a solidified intermediate article is obtained from which the solvent and the porogen agent are then removed rendering the porous article of the invention having the desired and controlled porous morphology. Said article is a further aspect of the present invention as already above mentioned.

Removal of solvent and porogen agent is carried out by extracting or leaching with another solvent, hereinafter referred to as the non-solvent, since it cannot dissolve the PEEK-type polymer. PEEK-type polymers are known to be insoluble in many common organic solvents which thus do not affect the article properties.

Said non-solvent can be one or more liquid solvents, has to be miscible with the solvent and capable of dissolving both the solvent and/or the porogen agent. The non-solvent can thus be readily determined by the skilled person in each case. For instance the solvent benzophenone can be removed with ethanol; the solvent pentafluorophenol with distilled water and phenilsulfone with acetone. The porogen agent, such as a salt-type agent may be removed for instance with distilled water.

The leaching step can be carried out in a single contact extraction with a sufficient large volume of a non-solvent or by a sequence of several, at least two solvent extractions, with one or more liquid solvents.

Typically steps are carried out by submerging the intermediate articles in a non-solvent under stirring to facilitate extraction of the solvent and the porogen agent during times that may be vary from 5 minutes to 120 minutes, or several hours. The maximum extracting temperature is that at which the article is still not affected. The minimum temperature is that at which extraction occurs at a reasonable rate. Temperatures can thus be comprised within a wide range, typically comprised between 0 and 80°C, and more preferably at ambient temperature.

In a particular embodiment at least two different non-solvents, such as ethanol and distilled water, are used one after the other, in an alternating way. Each non-solvent can be used more than once.

Finally the resulting porous article can then be recovered. Said recovering of the article comprises for instance a freeze-drying step to completely remove the distilled water rendering the porous article of the invention.

According to the process of the invention the porosity, the pore distribution and the pore size and form of the pores corresponding to the macropores can be designed and controlled by selecting and determining variables such as the porogen agent/PEEK-type polymer ratio, the PEEK concentration, the porogen agent particle size and form, and the cooling temperature.

Porosity refers to the volumetric void volume of the article and is defined as the fraction of the volume of voids over the total volume of a sample. The porosity of the articles of the present invention has been measured on a mercury porosimeter (AutoPore IV 9500 V1.09, Micrometrics). The porosity can vary within wide ranges, although typically porosities between 75-90% have been determined (see Examples 1 to 3). Pore size of an article can be estimated by several techniques including scanning electron microscopy (SEM).

The pore distributions have also been determined on a mercury porosimeter. Pore distributions A within the range between 50 and 500 µm can be narrow or broad depending on the characteristics of the porogen agent particles used. The obtained pore distributions were in accordance with the results observed in the SEM images (see for example Figure 5).

The pore distribution A and the pore size corresponding to the pores A can be controlled and varied as desired by the skilled person putting the present invention into practice within one article. In this sense the pore distribution A and the pore size corresponding to the pores A can be substantially homogeneous within a whole produced porous article due to the use of a substantially homogeneous porogen agent particle size which is homogeneously distributed within the mixture of PEEK-type polymer and porogen agent obtained after step c). Alternatively the pore distribution A and the pore A size can be substantially heterogeneous within said whole article, due to the simultaneous use in the method of at least two different porogen agent particles differing at least in their size distributions. Said at least two different porogen agent particles presenting different sizes, may be used in the process homogeneously distributed within the whole article to be obtained or may be heterogeneously distributed within the article. According to the latter embodiment, particles having a certain size may be distributed in a first area of the article to be obtained, and particles of the different size may be distributed in a different second area. According to a different embodiment, particles having a certain size are distributed in a certain area of the article to be obtained, for instance the lower part of an article, particles of the different size are located in a different area for instance the upper part of said article, and mixtures of both particles are located in a still different area (in the middle part). In this way a gradient of porosity may be designed within an article. The different areas above referred to can also be at least a first inner part and a second outer part. Thus, it is to be understood that all different possibilities of combing all different particles sizes and using them by distributing them in certain areas of the obtained article are contemplated according to the present invention. It is also to be understood that the process of the present invention contemplates controlling and varying the pore distribution A and the pore size corresponding to the pores A as explained within one article, in combination with the simultaneous use of at least one bioactive ceramic as above exposed.

The process of the present invention provides articles with homogeneous and/or heterogeneous pore size distributions within the whole article.

The resulting variety of PEEK-type polymer porous articles of the present invention may be used for many different applications, such as tissue engineering scaffolds, due to the PEEK type polymer biocompatibility, cell culture matrices, controlled release matrices, wound dressings, separation membranes, column fillers of chromatography, filters, packaging and insulating materials, among others.

Articles may according to their intended use present different forms such as membranes, cylinders, prisms, etc. Moreover, once obtained, porous articles may be further processed if necessary, according to conventional techniques, such as cutting, to further adjust its shape or size to the desired concrete application.

According to a particular embodiment the porous articles are used in applications such as tissue engineering scaffolds due to its advantageous morphology. The pores A and B facilitate the entrance of cells and the growing of bone tissue, and the pores C facilitate the absorption of proteins, facilitate the transport of nutrients, and enhance the adhesion, proliferation and cell differentiation due to the nanometric topography, which is similar to that presented by the bone. The combination of at least these different pore distributions has been shown to be essential for the success of the porous article of the invention as a porous implant and/or scaffold.

Depending on the intended use of an article, parameters such as porosity, pore size distribution, size and shape of said article, its composition, for instance the presence and the concentration of a certain bioactive ceramic, among others are well-designed and controlled.

Thus in another aspect the present invention relates to the use of the porous article of the invention as a porous implant and/or scaffold.

The foregoing is illustrative of the present invention. This invention however is not limited to the following precise embodiments described herein, but encompasses all equivalent modifications.

### EXAMPLES

### Process for the production of an article comprising polyetheretherketone and its characterization

The characterization of the articles was carried out as follows:
Porosity, average pore size and pore size distribution were measured on a mercury porosimeter (AutoPore IV 9500 V1.09, Micromeritics).

Microstructures of the samples were valuated by scanning electron microscopy (SEM). The articles were fractured in liquid nitrogen and then sputtered with gold to observe the morphology of the cross section by SEM. (JEOL JSM 5910-LV (20 kV)). The image analysis coupled with Energy Dispersive Spectrometry (EDS) analysis on a SEM was applied to the characterization of the elemental composition of the particles observed in the SEM images.

### Example 1:

Firstly, sodium chloride (Sigma Aldrich) particles of size between 80-120µm were sifted with standard sieves and collected to obtain the desired sizes.

Next, 800 mg of polyetheretherketone (PEEK) (VESTAKEEP, LATI) and 3200 mg of benzophenone (BF) (Panreac) were added into a 10mL glass vial. N₂ was bubbled into the vial for 5 min. and then the vial was sealed with a screw cap. The glass vial was introduced in an oil bath and heated up to 285° C. The mixture was vigorously stirred until PEEK completely dissolved in BF, forming a homogeneous and transparent solution. Once the polymer was dissolved, 2 g of sieved salt particles were added to the PEEK/BF solution and the dispersion was maintained under stirring at 285° C for 30 minutes.

After this process, the glass vial was removed from the oil bath and maintained at room temperature overnight without stirring. The solidified PEEK/BF/salt intermediate was immersed in 50mL ethanol on a shaker at 100 r.p.m. at room temperature for 24 h (the ethanol was changed every 12 h) to leach out the BF. Then, the ethanol was removed and the sample immersed in 50mL distilled water on a shaker at 100 r.p.m. at room temperature for 24 h (the water was changed every 12 h) to leach out the salt. These two processes were alternately carried out during 8 days. Finally, the porous PEEK sample was freeze-dried to completely remove the distilled water and the porous PEEK article was obtained. The spaces originally occupied by the solvent and the porogen particles became pores A, B and C in the PEEK article.

### Results

The porosimeter results showed that the porosity of the article was 84%. It exhibited multimodal (trimodal) distribution of pores. One pore size distribution A was centered at 95 µm due to the extraction of porogen particles; another pore size distribution B was centered at 5 µm due to the opening created by the bonding of two adjacent salt particles. And a pore size distribution C, smaller than 1 µm, due to the extraction benzophenone.

These results were in good agreement with those observed in the SEM pictures (Figure 1). Larger pores A retaining the shapes of the original porogen particles, were observed. Moreover, in the wall of those pores, micro- and nanopores C were detected due to the benzophenone elimination. And finally, pores B around 5 µm were appreciated that match to the size of the openings between the pores A.

### Example 2:

Firstly, sodium chloride (Sigma Aldrich) particles of size between 120-180µm were sifted with standard sieves and collected to obtain the desired sizes.

Next, 80 mg of hydroxyapatite (HA) (Plasma Biotal) and 3200 mg of benzophenone (BF) (Panreac) were added into a 10mL glass vial, N₂ was bubbled into the vial for 5 min. and then the vial was sealed with a screw cap and the mixture was sonicated at 60° C for 30 minutes. Once the HA was dispersed in BF, 800 mg of polyetheretherketone (PEEK) (VESTAKEEP, LATI) was added and the glass vial was transferred to an oil bath and heated at 285 °C. The mixture was vigorously stirred until PEEK was completely dissolved. When the polymer was dissolved, 2 g of sieved salt particles were added to the PEEK /HA/BF dispersion and the solution was maintained under stirring at 285° C for 30 minutes.

After this process, the glass vial was removed from the oil bath and maintained at room temperature overnight without stirring. The solidified PEEK/HA/BF/salt intermediate article was immersed in 50mL ethanol on a shaker at 100 r.p.m. at room temperature for 24 h (the ethanol was changed every 12 h) to leach out the BF. Then, the ethanol was removed and the sample immersed in 50mL distilled water on a shaker at 100 r.p.m. at room temperature for 24 h (the water was changed every 12 h) to leach out the salt. These two processes were alternately carried out during 8 days. Finally, the porous PEEK/HA article was freeze-dried to completely remove the distilled water rendering an article according to the invention.

### Results

The porosimeter results showed that the porosity of the article was 86%. It exhibited multimodal (trimodal) distribution of pores. One pore size distribution A was centered at 187µm due to the extraction of porogen particles; another one B was centered at 62µm due to the opening created by the bonding of two adjacent salt particles. And pore size distribution C smaller than 1 µm due to the extraction benzophenone.

Larger pores A can be observed retaining the shapes of the original porogen particles, (Figure 2a). Inter pore opening size of about 60 µm was detected in the bonding of the pores A. Moreover, in the wall of those pores, micro and nanopores C were detected due to the benzophenone elimination (Figure 2b,c,d).

HA particles can be also appreciated, with a size around 3 µm, distributed throughout the whole article (Figure. 2b, c, e). These particles were successfully integrated into the matrix with no visible agglomeration formation.

From the EDS characterization (Figure 3) can be confirmed that the particles observed in the SEM images were, basically, composed by calcium and phosphorous elements, two of the mayor components of HA.

### Example 3:

Firstly, sodium chloride (Sigma Aldrich) particles of size between 80-120µm were sifted with standard sieves and collected to obtain the desired sizes.

Next, 400 mg of polyetheretherketone (PEEK) (VESTAKEEP, LATI) and 3600 mg of pentafluorophenol (PF) (Panreac) were added into a 10mL glass vial. N₂ was bubbled into the vial for 5 min. and then the vial was sealed with a screw cap. The glass vial was introduced in an oil bath and heated up to 150° C. The mixture was vigorously stirred until PEEK completely dissolved in PF, forming a homogeneous and transparent solution. Once the polymer was dissolved, 2 g of sieved salt particles were added to the PEEK/PF solution and the dispersion was maintained under stirring at 150° C for 30 minutes.

After this process, the glass vial was removed from the oil bath and maintained at room temperature overnight without stirring. The solidified PEEK/PF/salt intermediate was immersed in 50mL distilled water on a shaker at 100 r.p.m. at room temperature for 8 days (the distilled water was changed every 12 h) to leach out the PF and porogen particles. Finally, the porous PEEK sample was freeze-dried to completely remove the distilled water. The spaces originally occupied by the solvent and porogen particles became pores in the porous PEEK article.

### Results

The porosimeter results showed that the porosity of the article was 83%. It exhibited multimodal (trimodal) distribution of pores. One pore size distribution A was centered at 73µm due to the extraction of porogen particles; another pore size distribution B was centered at 1.5µm corresponded to the bonding areas between the porogen particles. And the last pore distribution C was <1 µm due to the extraction benzophenone.

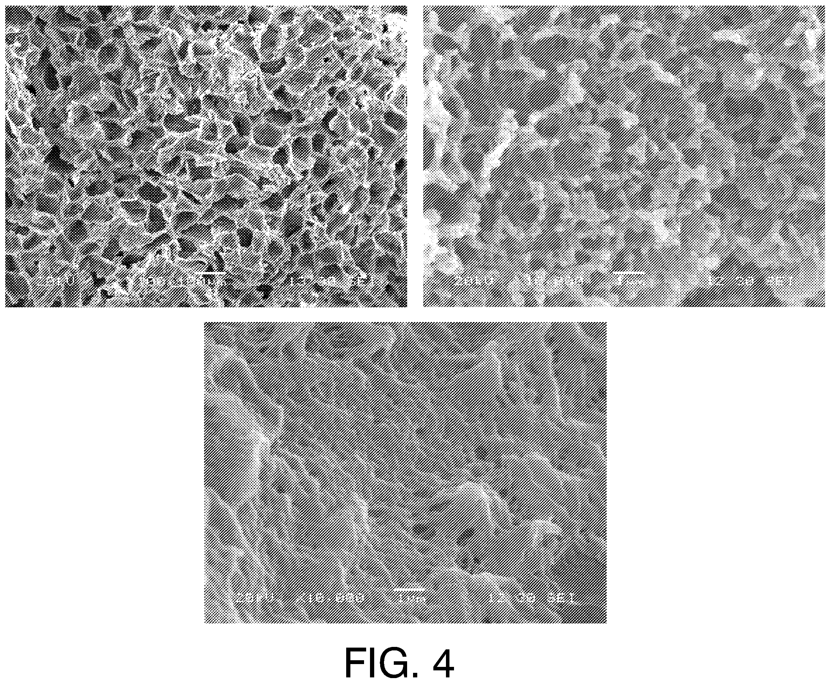

These results were in good agreement with those observed in the SEM pictures (Figure 4). Larger pores A retaining the shapes of the original porogen particles, were observed. Moreover, in the wall of those pores A and B, micro and nanopores C were detected due to the size of the opening between the pores A and the benzophenone elimination).

## Claims

1. A porous PEEK-type polymer article comprising a porous PEEK-type polymer structure and presenting at least a trimodal pore distribution as follows:
(i). - a pore distribution A corresponding to pores A of an average size between 50 µm and 500 µm which are interconnected throughout the whole article;
(ii). - a pore distribution B corresponding to the voids between adjacent pores A of an average size between 5 µm and 70 µm referred to as pores B;
(iii). - a pore distribution C corresponding to pores C of an average size of 5 µm or less which are located in the walls of the pores A and pores B.

2. An article according to claim 1, wherein the pores occupy between 75 and 90 % of the total volume of the article.

3. An article according to any one of claims 1 to 2, further comprising bioactive ceramic particles integrated in the porous PEEK-type polymer structure and distributed throughout the whole article.

4. A process for the production of a porous PEEK-type polymer article according to any one of claims 1 to 3, comprising the following steps:
a) contacting a PEEK-type polymer with a composition comprising at least an organic solvent,
b) heating at a temperature at which the PEEK-type polymer is dissolved,
c) adding at least a porogen agent, in an amount comprised between 50 to 90% wt in respect of the mixture PEEK-type polymer-solvent weight,
d) cooling the mixture obtained in c) at a temperature at least equal or lower than the temperature at which the PEEK-type polymer precipitates,
e) forming said cooled mixture into a shaped intermediate article,
f) removing the organic solvent and the porogen agent,
g) recovering the article comprising a PEEK-type polymer.

5. A process according to claim 4, wherein the organic solvent presents at least one six membered ring structure and a boiling temperature between 150°C and 400°C and is capable of dissolving at least 10% of the PEEK-type polymer present at the article forming temperature.

6. A process according to claim 5, wherein the organic solvent is selected from the group consisting of benzophenone, pentafluorophenol, phenilsulphone, 2-phenilphenol, dimethylphthalate, phenylbenzoate, ethyl-4-hydroxybezoate, n-cyclohexyl-2-pyrrolidone, preferably benzophenone or pentafluorophenol.

7. A process according to any one of claims 4 to 6, wherein the composition comprising at least an organic solvent further comprises a bioactive ceramic.

8. A process according to claim 7, wherein said bioactive ceramic is selected from the group consisting of calcium phosphates, preferably tricalcium phosphate, hydroxyapatite, biphasic calcium phosphate, and their mixtures.

9. A process according to any of claims 4 to 8, wherein said porogen agent is selected from the group of sugar-type porogen agents, salt-type porogen agents and their mixtures.

10. A process according to claim 9, wherein said porogen agent is a salt-type porogen agent preferably selected from sodium chloride, sodium citrate, sodium tartrate, potassium chloride and their mixtures.

11. A process according to claim 9, wherein said porogen agent is a sugar-type porogen agent preferably selected from sacarose, galactose and their mixtures.

12. A process according to any one of claims 4 to 11, wherein forming the cooled mixture is carried out by placing said mixture in a mould presenting the shape and dimensions of the article to be obtained.

13. Use of the article according to any one of claims 1 to 3, as a porous implant and/or scaffold.

## Patentansprüche

1. Ein poröses PEEK-artiger Polymerartikel, umfassend eine poröse PEEK-artige Polymerstruktur und aufweisend zumindest eine trimodale Porenverteilung wie folgt:
(i). - eine Porenverteilung A korrespondierend zu Poren A mit einer Durchschnittsgröße zwischen 50 µm und 500 µm, die überall in dem gesamten Artikel verbunden sind;
(ii). - eine Porenverteilung B korrespondierend zu den Lücken zwischen benachbarten Poren A mit einer Durchschnittsgröße zwischen 5 µm und 70 µm, bezeichnet als Poren B;
(iii). - eine Porenverteilung C korrespondierend zu Poren C mit einer Durchschnittsgröße von 5 µm oder weniger, die in den Wänden zwischen den Poren A und den Poren B angeordnet sind.

2. Ein Artikel gemäß Anspruch 1, wobei die Poren zwischen 75 und 90 % des Gesamtvolumens des Artikels einnehmen.

3. Ein Artikel gemäß einem der Ansprüche 1 bis 2, weiter umfassend biologisch aktive Keramikpartikel, die in die poröse PEEK-artige Polymerstruktur integriert und über den gesamten Artikel verteilt sind.

4. Ein Prozess zur Herstellung eines porösen PEEK-artigen Polymerartikels gemäß einem der Ansprüche 1 bis 3, umfassend die folgenden Schritte:
a) Kontaktieren eines PEEK-artigen Polymers mit einer Zusammensetzung, umfassend zumindest ein organisches Lösungsmittel,
b) Heizen mit einer Temperatur, bei der das PEEK-artige Polymer aufgelöst ist,
c) Hinzufügen zumindest eines porenbildenden Wirkstoffs, in eine Menge umfassend zwischen 50 % bis 90 % mit Bezug auf das Gewicht der Mischung aus PEEK-artigem Polymer und Lösungsmittel,
d) Kühlen der in c) erhaltenen Mischung bei einer Temperatur zumindest gleich der oder niedriger als die Temperatur, bei der das PEEK-artige Polymer ausfällt,
e) Formen dieser gekühlten Mischung in einen geformten Zwischenartikel,
f) Entfernen des organischen Lösungsmittels und des porenbildenden Wirkstoffs,
g) Ausbringen des Artikels, umfassend ein PEEK-artiges Polymer.

5. Ein Prozess gemäß Anspruch 4, wobei das organische Lösungsmittel zumindest eine sechsgliedrige Ringstruktur und eine Siedetemperatur zwischen 150°C und 400°C aufzeigt und fähig ist, zumindest 10 % des PEEK-artigen Polymers vorhanden bei der Artikelformtemperatur aufzulösen.

6. Ein Prozess gemäß Anspruch 5, wobei das organische Lösungsmittel ausgewählt ist aus einer Gruppe bestehend aus Benzophenon, Pentafluorophenol, Phenylsulfon, 2-Phenylphenol, Dimethylphthalat, Phenylbenzoat, Ethyl-4-Hydroxybenzoat, N-Cyclohexyl-2-Pyrrolidone, vorzugsweise Benzophenon oder Pentafluorophenol.

7. Ein Prozess gemäß einem der Ansprüche 4 bis 6, wobei die Zusammensetzung, umfassend zumindest ein organisches Lösungsmittel, weiter eine biologisch aktive Keramik umfasst.

8. Ein Prozess gemäß Anspruch 7, wobei die biologisch aktive Keramik ausgewählt ist aus einer Gruppe bestehend aus Kalziumphosphat, vorzugsweise Trikalziumphosphat, Hydroxyapatit, biphasischem Kalciumphosphat und deren Mischungen.

9. Ein Prozess gemäß einem der Ansprüche 4 bis 8, wobei der porenbildende Wirkstoff ausgewählt ist aus einer Gruppe von zuckerartigen porenbildenden Wirkstoffen, salzartigen porenbildenden Wirkstoffen und deren Mischungen.

10. Ein Prozess gemäß Anspruch 9, wobei der porenbildende Wirkstoff ein salzartiger porenbildender Wirkstoff ist, vorzugsweise ausgewählt aus Natriumchlorid, Natriumcitrat, Natriumtartrat, Kaliumchlorid und deren Mischungen.

11. Ein Prozess gemäß Anspruch 9, wobei der porenbildende Wirkstoff ein zuckerartiger Wirkstoff ist, vorzugsweise ausgewählt aus Saccharose, Galaktose und deren Mischungen.

12. Ein Prozess gemäß einem der Ansprüche 4 bis 11, wobei das Formen der gekühlten Mischung ausgeführt wird durch Anordnen der Mischung in einer Gussform, aufzeigend die Form und Dimensionen des zu erhaltenden Artikels.

13. Verwendung des Artikels gemäß einem der Ansprüche 1 bis 3 als ein poröses Implantat und/oder ein Gerüst.

## Revendications

1. Article en polymère de type PEEK poreux comprenant une structure de polymère de type PEEK poreux et présentant au moins une distribution trimodale de pores de la manière suivante :
(i). - une distribution de pores A correspondant à des pores A d'une taille moyenne comprise entre 50 µm et 500 µm qui sont interconnectés dans l'ensemble de l'article ;
(ii). - une distribution des pores B correspondant aux vides entre des pores adjacents A d'une taille moyenne comprise entre 5 µm et 70 µm appelés pores B ;
(iii). - une distribution de pores C correspondant à des pores C d'une taille moyenne de 5 µm ou moins qui sont situés dans les parois des pores A et des pores B.

2. Article selon la revendication 1, où les pores occupent entre 75 et 90% du volume total de l'article.

3. Article selon l'une quelconque des revendications 1 à 2, comprenant en outre des particules céramiques bioactives intégrées dans la structure de polymère de type PEEK poreux et réparties dans tout l'article.

4. Procédé pour la production d'un article en polymère poreux de type PEEK selon l'une quelconque des revendications 1 à 3, comprenant les étapes suivantes :
a) mise en contact d'un polymère de type PEEK avec une composition comprenant au moins un solvant organique,
b) chauffage à une température à laquelle le polymère de type PEEK est dissous,
c) ajout d'au moins un agent porogène, dans une quantité comprise entre 50 et 90% en masse par rapport à la masse du mélange polymère-solvant de type PEEK,
d) refroidissement le mélange obtenu en c) à une température au moins égale ou inférieure à la température à laquelle le polymère de type PEEK précipite,
e) formation dudit mélange refroidi en un article intermédiaire formé,
f) enlèvement du solvant organique et de l'agent porogène,
g) récupération de l'article comprenant un polymère de type PEEK.

5. Procédé selon la revendication 4, où le solvant organique présente au moins une structure cyclique à six chaînons et une température d'ébullition comprise entre 150°C et 400°C et est capable de dissoudre au moins 10% du polymère de type PEEK présent à la température de formation de l'article.

6. Procédé selon la revendication 5, où le solvant organique est choisi dans le groupe constitué de benzophénone, pentafluorophénol, phénylsulfone, phénylphénol, diméthylphtalate, phénylbenzoate, éthyl-4-hydroxybenzoate, n-cyclohexyl-2-pyrrolidone, de préférence benzophénone ou pentafluorophénol.

7. Procédé selon l'une quelconque des revendications 4 à 6, où la composition comprenant au moins un solvant organique comprend en outre une céramique bioactive.

8. Procédé selon la revendication 7, où ladite céramique bioactive est choisie dans le groupe constitué par les phosphates de calcium, de préférence le phosphate tricalcique, l'hydroxyapatite, le phosphate de calcium biphasique et leurs mélanges.

9. Procédé selon l'une quelconque des revendications 4 à 8, où ledit agent porogène est choisi dans le groupe des agents porogènes du type sucre, des agents porogènes du type sel et leurs mélanges.

10. Procédé selon la revendication 9, où ledit agent porogène est un agent porogène de type sel de préférence choisi parmi le chlorure de sodium, le citrate de sodium, le tartrate de sodium, le chlorure de potassium et leurs mélanges.

11. Procédé selon la revendication 9, où ledit agent porogène est un agent porogène de type sucre, de préférence choisi parmi le saccarose, le galactose et leurs mélanges.

12. Procédé selon l'une quelconque des revendications 4 à 11, où la formation du mélange refroidi est réalisée en plaçant ledit mélange dans un moule présentant la forme et les dimensions de l'article à obtenir.

13. Utilisation de l'article selon l'une quelconque des revendications 1 à 3, en tant qu'implant poreux et/ou échafaudage.
